# EUROPÄISCHE PATENTSCHRIFT

(11) **EP 3 107 676 B1**
(45) Veröffentlichungstag und Bekanntmachung des Hinweises auf die Patenterteilung: **29.09.2021**
(21) Anmeldenummer: 15712777.0
(22) Anmeldetag: 19.02.2015
(51) Int. Cl.: B23C 5/20, B23C 5/06

(54) **INDEXIERBARER SCHNEIDEINSATZ UND FRÄSWERKZEUG**
INDEXABLE CUTTING INSERT AND MILLING TOOL
PLAQUETTE DE COUPE INDEXABLE ET FRAISE

(30) Priorität: 20.02.2014 AT 752014 U
(43) Veröffentlichungstag der Anmeldung: 28.12.2016
(73) Patentinhaber: Ceratizit Austria Gesellschaft m.b.H., 6600 Reutte (AT)
(72) Erfinder: BURTSCHER, Peter, A-6651 Häselgehr (AT)
(74) Vertreter: Ciesla, Dirk
(86) Internationale Anmeldenummer: PCT/AT2015/000027
(87) Internationale Veröffentlichungsnummer: WO 2015/123708

(56) Entgegenhaltungen:
- WO-A1-2005/065874
- WO-A1-2011/103609
- DE-A1-102009 035 754
- JP-A- 2005 169 511
- US-A1- 2010 202 839

## Beschreibung

Die vorliegende Erfindung betrifft einen indexierbaren Schneideinsatz zum Planfräsen mit großen Vorschüben und ein Fräswerkzeug zum Planfräsen mit großen Vorschüben mit einer Mehrzahl von solchen indexierbaren Schneideinsätzen.

Bei der Bearbeitung von insbesondere metallischen Werkstoffen durch Fräsen ist es üblich, Fräswerkzeuge einzusetzen, die einen relativ zähen Trägerkörper aufweisen, der z.B. aus Werkzeugstahl gefertigt sein kann, an dem eine Mehrzahl von Schneideinsätzen aus einem härteren, verschleißbeständigeren Material, insbesondere Hartmetall (cemented-carbide), Cermet oder Schneidkeramik, befestigt ist. Die Schneideinsätze weisen dabei die mit dem zu bearbeitenden Werkstück zerspanend in Eingriff gelangenden Schneidkanten auf. Es ist insbesondere bekannt, die Schneideinsätze als sogenannte indexierbare Schneideinsätze auszubilden, die eine Mehrzahl von nacheinander einsetzbaren, identischen Schneidkanten aufweisen. Das Indexieren oder "Weiterschalten" der individuellen Schneidkanten erfolgt dabei üblicherweise durch eine Drehung des jeweiligen Schneideinsatzes um eine oder mehrere Symmetrieachsen (z.B. um 90°, 120°, 180°, 240°, 270° oder andere Winkel).

Je nach Anwendungszweck kommen dabei unterschiedliche Geometrien der Fräswerkzeuge und der Schneideinsätze zum Einsatz. Ein Anwendungsbereich ist das Planfräsen mit großen Vorschüben, bei dem der Fräsvorgang mit relativ geringen Schnitttiefen aber relativ großen Vorschüben in der Richtung parallel zu der zu bearbeitenden Werkstückoberfläche durchgeführt wird.

Aufgrund des großen Vorschubs kommen bei derartigen Fräsoperationen relativ lange Schneidkantenabschnitte mit dem zu bearbeitenden Werkstück in Eingriff und es ist wichtig, eine möglichst ausgewogene Verteilung der Schnittkräfte an der Schneidkante bereitzustellen, um eine hohe Standzeit der Schneideinsätze zu erzielen.

JP S52-103081 A zeigt einen Fräser mit einem Trägerkörper, der mit einer Mehrzahl von auswechselbaren Schneideinsätzen bestückt ist, die jeweils zumindest abschnittsweise konvex ausgewölbte Schneidkantenabschnitte aufweisen.

DE 10 2009 035 754 A1 beschreibt einen Schneideinsatz gemäß dem Oberbegriff des Anspruchs 1, für ein Schneidwerkzeug zur spanenden Bearbeitung von Werkstücken, wobei das Schneidwerkzeug einen Halter mit einem Sitz zur Aufnahme des Schneideinsatzes aufweist. Der Schneideinsatz weist eine Schneidkante auf, die zur Erzielung hoher Vorschubgeschwindigkeiten und großer Schnitttiefen beim Hochvorschubfräsen abschnittsweise drei unterschiedliche Krümmungsradien aufweist.

US 2010/0202839 A1 beschreibt einen doppelseitigen Schneideinsatz mit einer Mehrzahl indexierbarer konvexer Schneidkanten, die über Ecken miteinander verbunden sind. Jede konvexe Schneidkante weist zwischen einem gekrümmten Schneidkantenbereich und einer Ecke einen primären, im Wesentlichen geraden Schneidkantenbereich auf.

WO 2005/065874 A1 beschreibt ein Schneidelement mit einer ersten Oberfläche und einer Seitenfläche, die in einem ersten Randbereich zusammentreffen. An dem Randbereich ist eine konvex gekrümmte Schneide ausgebildet, deren Krümmung in einem vorgegebenen Drehsinn um eine Drehachse streng monoton zunimmt.

Indexierbare Schneideinsätze aus Hartmetall (cemented carbide) oder Cermet werden üblicherweise in einem pulvermetallurgischen Herstellungsprozess aus Ausgangspulvern hergestellt, die entsprechend der gewünschten Zusammensetzung gemischt, in einer Matrize in die gewünschte Form gepresst und anschließend zu festen Körpern gesintert werden. Bei dem Sintern der gepressten Grünlinge tritt eine Verdichtung des Materials auf, die mit einer Schrumpfung einhergeht, wobei die Gleichmäßigkeit dieser Schrumpfung durch die Geometrie des Grünlings und die erreichte Druckverteilung beim Pressen stark beeinflusst wird. Es hat sich gezeigt, dass die erreichbare Standzeit bei pulvermetallurgisch hergestellten Schneideinsätzen stark von der erfolgreichen Kontrolle des pulvermetallurgischen Herstellungsprozesses abhängt.

Es ist Aufgabe der vorliegenden Erfindung, einen verbesserten indexierbaren Schneideinsatz zum Planfräsen mit großen Vorschüben und ein verbessertes Fräswerkzeug zum Planfräsen mit großen Vorschüben bereitzustellen, mit denen ein zuverlässiger Fräsvorgang und eine lange Standzeit bei pulvermetallurgisch hergestellten indexierbaren Schneideinsätzen ermöglicht werden.

Die Aufgabe wird durch einen indexierbaren Schneideinsatz zum Planfräsen mit großen Vorschüben nach Anspruch 1 gelöst. Vorteilhafte Weiterbildungen sind in den abhängigen Ansprüchen angegeben.

Durch den kontinuierlich konvex gekrümmten Verlauf der Schneidkanten wird eine besonders ausgewogene Verteilung der Schnittkräfte beim Fräsen mit großen Vorschüben erzielt. In Kombination mit der sich nur über einen Teil der Höhe der Seitenfläche erstreckenden Hauptfreiflächen, die über eine Stufe in nach innen rückversetzte Sekundärflächen übergehen, wird eine besonders homogene Druckverteilung beim Pressen während des pulvermetallurgischen Herstellungsprozesses ermöglicht. Die homogene Druckverteilung resultiert in einem sehr gleichmäßigen Verzugsverhalten beim Sinterprozess, sodass der indexierbare Schneideinsatz nach dem Durchlaufen des Herstellungsprozesses eine hohe Standzeit beim Planfräsen mit großen Vorschüben erzielt.

Gemäß einer Weiterbildung weist der Schneideinsatz eine in Aufsicht auf die Oberseite im Wesentlichen quadratische Grundform mit vier Schneidecken und vier die Schneidecken verbindenden Schneidkanten auf. In diesem Fall werden eine robuste Ausgestaltung des Schneideinsatzes und gleichzeitig eine relativ große Anzahl von individuell einsetzbaren Schneidkanten erzielt.

Gemäß einer Weiterbildung erstreckt sich die Hauptfreifläche in Richtung zu der Unterseite über weniger als 50 % der Höhe der Seitenfläche, bevorzugt über weniger als 40 % der Höhe der Seitenfläche. In diesem Fall wird erreicht, dass die Druckverteilung beim Pressen und der Sinterverzug sehr genau kontrolliert werden können, sodass eine hohe Standzeit ermöglicht wird. Ferner wird in diesem Fall ein niedriger Freiflächenverschleiß erzielt.

Gemäß einer Weiterbildung weisen die Schneidkanten einen im Wesentlichen ellipsensegmentförmigen Verlauf auf. In diesem Fall wird eine besonders ausgewogene Verteilung der Schnittkräfte über die Schneidkante erreicht.

Bevorzugt ist entlang der Schneidkanten und Schneidecken an der Oberseite eine Fase ausgebildet. In diesem Fall ist die Stabilität der Schneidkante durch die Fase zusätzlich erhöht. Bevorzugt weist die Fase eine Breite zwischen 0,1 mm und 0,4 mm auf.

Gemäß einer Weiterbildung verläuft die Fase im Bereich der Schneideecke unter einem ersten Fasenwinkel im Bereich zwischen -10° und -20° zu einer Haupterstreckungsrichtung der Unterseite und im Bereich der Mitte der Schneidkante unter einem flacheren zweiten Fasenwinkel zwischen 0° und -10°. Der angegebene Fasenwinkel wird dabei zwischen der Fase und der Haupterstreckungsrichtung der Unterseite bestimmt, wobei ein positiver Wert für den Fasenwinkel einer zum Zentrum des Schneideinsatzes hin abfallenden Fase entspricht und ein negativer Wert einer nach außen hin abfallenden Fase. Durch den besonderen angegebenen Verlauf der Fase wird eine besonders hohe Stabilität der Schneidkante bei verschiedenen Zerspanungsparametern erzielt. Die Fase fällt somit im Bereich der Schneidecke relativ stark nach außen hin ab, wodurch die Schneidecke eine besondere Verstärkung erfährt, und zur Mitte der Schneidkante hin fällt die Fase nach außen hin weniger stark ab oder nimmt sogar eine neutrale Ausrichtung an. In dieser Weise werden eine besonders vorteilhafte Verteilung der Schnittkräfte und eine hohe Stabilität des Schneideinsatzes ermöglicht. Gemäß einer Weiterbildung ist ein an die Schneidecke angrenzender Bereich der Schneidkante als ein Übergangsbereich ausgebildet, in dem der Winkel der Fase von dem ersten Fasenwinkel auf den zweiten Fasenwinkel übergeht.

Gemäß einer Weiterbildung sind die Seitenflächen über ihre gesamte Höhe unter einem schneideinsatzspezifischen Freiwinkel von ≥ 0° ausgebildet. Unter dem "schneideinsatzspezifischen Freiwinkel" wird dabei der Freiwinkel verstanden, der sich aus der Geometrie des Schneideinsatzes selbst bestimmt. Es ist zu beachten, dass der bei der Zerspanung resultierende effektive Freiwinkel zusätzlich durch die Einbaulage des Schneideinsatzes an der Schneideinsatzaufnahme des Fräswerkzeugs mitbestimmt wird. Die Ausgestaltung des schneideinsatzspezifischen Freiwinkels derart, dass dieser über die gesamte Höhe des Schneideinsatzes nicht kleiner als 0° wird, ermöglicht eine besonders einfache und zuverlässige Herstellung des Schneideinsatzes in einem pulvermetallurgischen Herstellungsprozess, bei dem sich eine homogene Druckverteilung beim Pressen zuverlässig erzielen lässt.

Gemäß einer Ausgestaltung ist die Unterseite als eine ebene Auflagefläche ausgebildet. In diesem Fall ist eine besonders zuverlässige Abstützung und genaue Orientierung des Schneideinsatzes im Betrieb ermöglicht.

Gemäß einer bevorzugten Weiterbildung ist zumindest ein Teil der Sekundärflächen zumindest bereichsweise als ebene Anlageflächen ausgebildet. In diesem Fall wird eine besonders stabile und zuverlässige Positionierung des Schneideinsatzes ermöglicht.

Gemäß einer bevorzugten Ausgestaltung weist der Schneideinsatz ein den Schneideinsatz von der Oberseite zu der Unterseite durchdringendes Durchgangsloch zum Aufnehmen einer Befestigungsschraube auf.

Insbesondere bei einem Schneideinsatz, der in einem pulvermetallurgischen Herstellungsprozess gefertigt ist, wirken sich die erfindungsgemäßen Merkmale sehr positiv auf die erreichbare Standzeit aus.

Gemäß einer Weiterbildung kann die Oberseite mit zumindest einer Spanleitstufe versehen sein. Es kann dabei z.B. eine sich im Wesentlichen über den gesamten Verlauf der Schneidkante erstreckende Spanleitstufe vorgesehen sein oder es können z.B. auch in verschiedenen Bereichen der Oberseite jeweils Spanleitstufen vorgesehen sein. Die Spanleitstufen können dabei jeweils eine oder mehrere Vertiefungen und/oder eine oder mehrere Vorsprünge aufweisen, die den bei der Zerspanung gebildeten Span leiten, verformen und/oder brechen.

Die Aufgabe wird ferner durch ein Fräswerkzeug zum Planfräsen mit großen Vorschüben gemäß Anspruch 15 gelöst.

Das Fräswerkzeug weist einen Halter mit einer Mehrzahl von Schneideinsatzaufnahmen und eine Mehrzahl von an den Schneideinsatzaufnahmen befestigten Schneideinsätzen der zuvor angegebenen Art auf.

Weitere Vorteile und Weiterbildungen ergeben sich aus der nachfolgenden Beschreibung eines Ausführungsbeispiels unter Bezugnahme auf die beiliegenden Zeichnungen.

Von den Figuren zeigen:
- Fig. 1:: eine perspektivische Darstellung eines indexierbaren Schneideinsatzes gemäß einer Ausführungsform;
- Fig. 2:: eine Seitendarstellung des indexierbaren Schneideinsatzes gemäß der Ausführungsform;
- Fig. 3:: eine Aufsicht auf den indexierbaren Schneideinsatz gemäß der Ausführungsform;
- Fig. 4:: eine Schnittdarstellung entlang der Linie B-B von Fig. 3;
- Fig. 5:: eine weitere perspektivische Darstellung des indexierbaren Schneideinsatzes gemäß der Ausführungsform;
- Fig. 6:: eine vergrößerte Darstellung eines Ausschnitts des oberen rechten Bereichs von Fig. 5;
- Fig. 7:: eine vergrößerte Darstellung eines Teils des indexierbaren Schneideinsatzes im Bereich einer Schneidecke;
- Fig. 8:: eine noch weiter vergrößerte Detaildarstellung von Fig. 7; und
- Fig. 9:: eine perspektivische Darstellung eines Fräswerkzeugs zum Planfräsen mit großen Vorschüben gemäß der Ausführungsform.

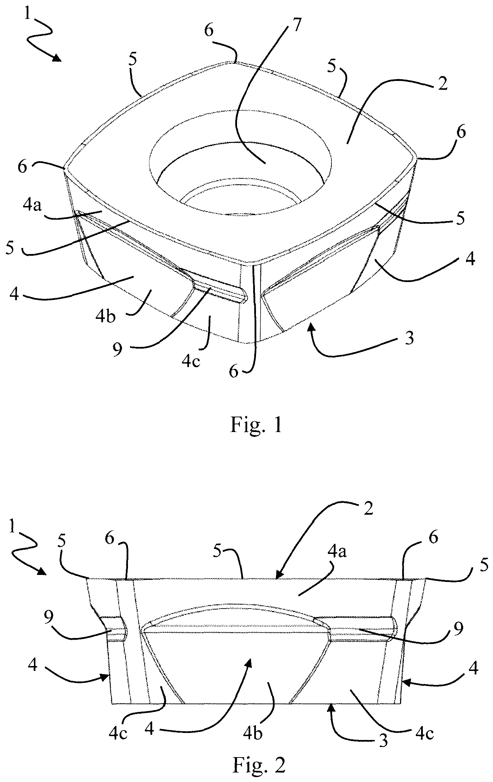

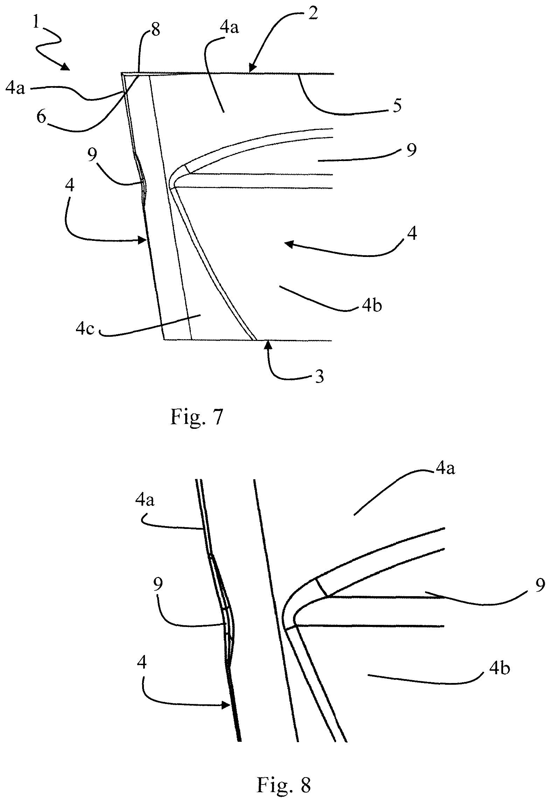

Eine Ausführungsform wird im Folgenden unter Bezug auf die Figuren eingehender beschrieben. Zunächst wird unter Bezug auf die Fig. 1 bis Fig. 8 ein indexierbarer Schneideinsatz 1 eingehender beschrieben.

Der indexierbare Schneideinsatz 1 ist zum Planfräsen mit großen Vorschüben ausgebildet und ist in einem pulvermetallurgischen Herstellungsprozess aus entsprechenden Ausgangspulvern hergestellt. Der Schneideinsatz 1 ist dabei insbesondere aus Hartmetall (cemented carbide) oder Cermet gefertigt, bevorzugt aus Hartmetall. Bei Hartmetall und Cermet handelt es sich um Werkstoffe, bei denen harte Teilchen in einer Bindermatrix aus einem duktileren Material eingebettet sind. In dem Fall von Hartmetall kann die Bindermatrix dabei insbesondere im Wesentlichen Kobalt (Co) aufweisen oder durch Kobalt gebildet sein und die harten Teilchen können insbesondere Wolframkarbid (WC) aufweisen und gegebenenfalls in geringeren Mengen z.B. kubische Karbide der Gruppen 4, 5 oder 6 des Periodensystems der Elemente. Es sind jedoch auch andere Binderbestandteile und/oder harte Teilchen möglich.

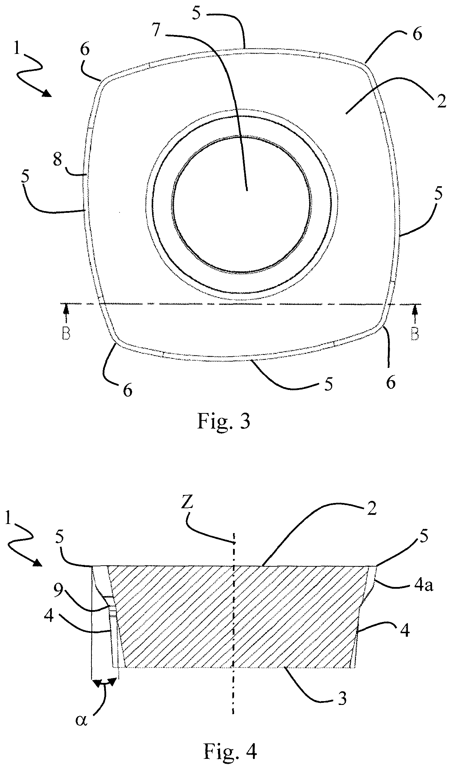

Der indexierbare Schneideinsatz 1 gemäß der Ausführungsform weist eine Oberseite 2, eine als Auflagefläche ausgebildete, im Wesentlichen ebene Unterseite 3 und eine Mehrzahl von sich zwischen der Oberseite 2 und der Unterseite 3 erstreckenden Seitenflächen 4 auf. Der Schneideinsatz 1 weist in Aufsicht auf die Oberseite 2 eine im Wesentlichen quadratische Grundform mit abgerundeten Ecken und konvex ausgewölbten Seiten auf, wie insbesondere in Fig. 3 zu sehen ist. Der Schneideinsatz 1 gemäß der Ausführungsform ist als sogenannter einseitiger Schneideinsatz ausgebildet, bei dem nur an dem Übergang zwischen den Seitenflächen 4 und der Oberseite 2 Schneidkanten 5 und Schneidecken 6 ausgebildet sind. Die Unterseite 3 des Schneideinsatzes 1 weist ferner einen geringeren Außenumfang als die Oberseite 2 auf, sodass der Schneideinsatz 1 eine sogenannte "positive" Formgebung aufweist, wobei sich die Bezeichnung "positiv" auf die realisierten schneideinsatzspezifischen Freiwinkel zurückführen lässt.

Die Schneidecken 6 sind abgerundet ausgebildet und weisen, in Aufsicht auf die Oberseite 2 betrachtet, eine kreissegmentförmige Ausgestaltung mit einem vorgegebenen Radius auf. Die Schneidkanten 5 erstrecken sich jeweils von einer Schneidecke 6 bis zu einer benachbarten Schneidecke 6 zwischen den Schneidecken 6. Die Schneidkanten 5 weisen, in Aufsicht auf die Oberseite 2 betrachtet, jeweils über ihre gesamte Erstreckung einen gekrümmten, konvex ausgewölbten Verlauf auf, der frei von geraden Abschnitten ist. Bei der speziell dargestellten Ausführungsform weisen die Schneidkanten 5 einen besonders bevorzugten ellipsensegmentförmigen Verlauf auf, bei dem sich die jeweilige Schneidkante 5 entlang der Kontur eines Segments einer Ellipse erstreckt. Es sind jedoch auch von dieser speziellen Formgebung abweichende Formen möglich.

Der indexierbare Schneideinsatz 1 gemäß der Ausführungsform weist somit vier identisch ausgebildete Schneidkanten 5 und vier identisch ausgebildete Schneidecken 6 auf. Ein Durchgangsloch 7 durchdringt den Schneideinsatz 1 von der Oberseite 2 zu der Unterseite 3 und verläuft koaxial zu einer Symmetrieachse Z. Das Durchgangsloch 7 ist dazu ausgebildet, eine Befestigungsschraube aufzunehmen, mit der der Schneideinsatz 1 an einer Schneideinsatzaufnahme 21 eines Halters 20 eines Fräswerkzeugs 100 befestigt werden kann, wie noch eingehender beschrieben wird. Der Schneideinsatz 1 weist eine vierzählige Rotationssymmetrie bzgl. der Symmetrieachse Z auf, sodass der Schneideinsatz 1 durch Drehung um 90°, 180°, 270° und 360° um die Symmetrieachse Z in dieselbe Form übergeführt werden kann. Es kann somit zur Indexierung des Schneideinsatzes 1 nach einer Abnutzung einer ersten der Schneidkanten 5, die nächste Schneidkante 5 durch Drehung des Schneideinsatzes 1 um die Symmetrieachse Z in eine aktive Schneidposition gebracht werden.

Die Oberseite 2 des Schneideinsatzes 1 ist zumindest in den an die Schneidkanten 5 und Schneidecken 6 angrenzenden Bereichen als Spanfläche ausgebildet, auf der die bei der Fräsbearbeitung gebildeten Späne des bearbeiteten Werkstücks abgeführt werden. Obwohl in den Figuren schematisch eine im Wesentlichen ebene Ausgestaltung der Oberseite 2 dargestellt ist, kann die Oberseite 2 bevorzugt in an sich bekannter Weise mit Spanleitstufen versehen sein, d.h. mit Vertiefungen und Erhöhungen, die eine spanleitende, spanformende und/oder spanbrechende Wirkung haben. Je nach zu bearbeitendem Material sind unterschiedliche Ausgestaltungen der Spanleitstufen möglich, worauf im Folgenden nicht eingehender eingegangen wird. Die Oberseite 2 kann dabei insbesondere derart ausgebildet sein, dass sie in einem unmittelbar an eine Fase 8 an der Schneidkante 5 bzw. Schneidecke 6 angrenzenden Bereich mit zunehmendem Abstand von der Schneidkante 5 bzw. Schneidecke 6 zunächst in Richtung der Unterseite 3 abfällt und nach Durchlaufen eines Spangrundes in Richtung zu der Symmetrieachse Z wieder unter Ausbildung eines Spanrückens ansteigt.

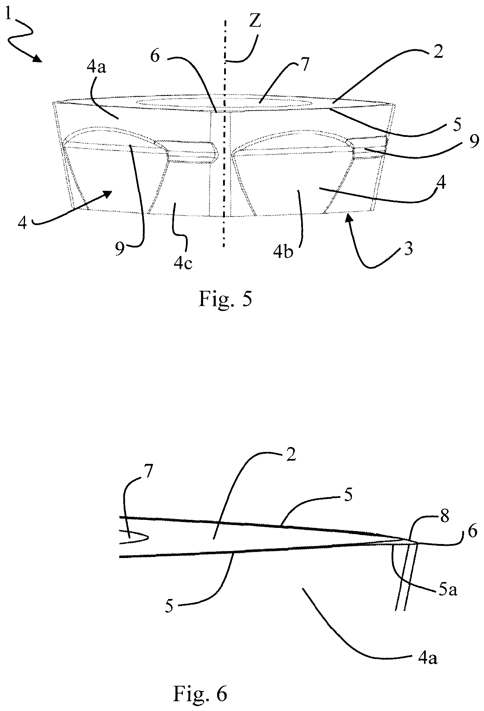

Entlang der Schneidkanten 5 und der Schneidecken 6 ist der Schneideinsatz 1 mit einer umlaufenden, die Schneidkanten 5 und Schneidecken 6 verstärkenden Fase 8 versehen. Die Fase 8 hat dabei bevorzugt eine Breite in der Richtung senkrecht zu der Schneidkante 5 bzw. Schneidecke 6 zwischen 0,1 mm und 0,4 mm. Wie insbesondere in Fig. 6 zu sehen ist, ist die Fase 8 dabei in dem Bereich der Schneidecke 6 derart ausgebildet, dass sie nach außen (d.h. mit zunehmendem Abstand vom Zentrum des Schneideinsatzes 1) abfällt. Die Fase 8 verläuft dabei im Bereich der Schneidecken 6 unter einem im Wesentlichen konstanten ersten Fasenwinkel im Bereich zwischen -10° und -20°, z.B. -15°. Wie in den Fig. 6 und Fig. 2 zu sehen ist, verläuft die Fase im Bereich der Schneidkanten 5 unter einem flacheren zweiten Fasenwinkel, der bei der Ausführungsform zwischen 0° und -10° beträgt, z.B. 0°. Wie in Fig. 6 zu erkennen ist, geht in einem unmittelbar an die Schneidecke 6 angrenzenden Übergangsabschnitt 5a der Schneidkante 5 der Winkel der Fase 8 kontinuierlich von dem ersten Fasenwinkel auf den flacheren zweiten Fasenwinkel über. Der Übergangsabschnitt 5a ist dabei relativ kurz und weist bevorzugt eine Länge auf, die in dem Bereich des 1,0-fachen bis 1,8-fachen des Krümmungsradius der Schneidecke 6 entspricht. In dem restlichen Bereich der Schneidkante 5 weist die Fase 8 bevorzugt im Wesentlichen konstant den flacheren zweiten Fasenwinkel auf.

Die Seitenflächen 4 sind in dem unmittelbar an die Schneidkanten 5 angrenzenden Bereich als Hauptfreiflächen 4a ausgebildet, die sich konvex gekrümmt durchgängig von einer Schneidecke 6 entlang der jeweiligen Schneidkante 5 bis zu der benachbarten Schneidecke 6 erstrecken. Die Hauptfreiflächen 4a erstrecken sich dabei in Richtung zu der Unterseite 3 nicht über die gesamte Höhe der Seitenflächen 4, sondern nur über einen Teil der Höhe der Seitenflächen 4, bevorzugt über weniger als 50 % der Höhe der Seitenflächen 4, mehr bevorzugt über weniger als 40 % der Höhe der Seitenflächen 4.

In Richtung zu der Unterseite 3 gehen die Hauptfreiflächen 4a in Form einer zu dem Zentrum des Schneideinsatzes 1 rückspringenden Stufe 9 gestuft in nach innen rückversetzte Sekundärflächen 4b und 4c über. Die Seitenflächen 4 des Schneideinsatzes 1 weisen dabei über die gesamte Höhe der Sekundärflächen 4b und 4c einen geringeren Außenumfang auf als im Bereich der Hauptfreiflächen 4a. Die Seitenflächen 4 sind dabei bevorzugt derart ausgebildet, dass die Seitenflächen 4 überall, d.h. über ihre gesamte Höhe und Länge, einen schneideinsatzspezifischen Freiwinkel α aufweisen, für den gilt α ≥ 0. Mit anderen Worten verlaufen die Seitenflächen 4 in allen Bereichen in Richtung zu der Unterseite 3 jeweils nach innen geneigt bzw. verlaufen höchstens abschnittsweise parallel zu der Symmetrieachse Z, sodass die Seitenflächen 4 frei von Hinterschneidungen ausgebildet sind. Es ist somit auch im Bereich der rückspringenden Stufe 9 keine Hinterschneidung ausgebildet.

Durch die beschriebene Ausgestaltung der Seitenflächen 4 mit der einen Rücksprung ausbildenden Stufe 9 wird bei der pulvermetallurgischen Herstellung des indexierbaren Schneideinsatzes 1 gemäß der Ausführungsform eine homogene Druckverteilung bei dem Pressvorgang erzielt. Dies wiederum resultiert in einem gut kontrollierbaren Schrumpfungsverhalten beim anschließenden Sintern des gepressten Grünlings, sodass eine besonders gute Stabilität der Schneidkanten 5 und der Hauptfreiflächen 4a erreicht wird.

Die Seitenflächen 4 weisen bei der Ausführungsform jeweils eine erste Sekundärfläche 4b auf, die als Anlagefläche zur Positionierung des Schneideinsatzes 1 in einer Schneideinsatzaufnahme eines Fräswerkzeugs ausgebildet ist. Die erste Sekundärfläche 4b ist dabei zumindest im Wesentlichen eben ausgebildet. Die Seitenflächen 4 weisen ferner weitere Sekundärflächen 4c mit einer konvex gekrümmten Form auf. Im Bereich der Schneidecken 6 erstrecken sich die Seitenflächen 4 jeweils gekrümmt bis zu dem Übergang zwischen den Seitenflächen 4 und der Unterseite 3.

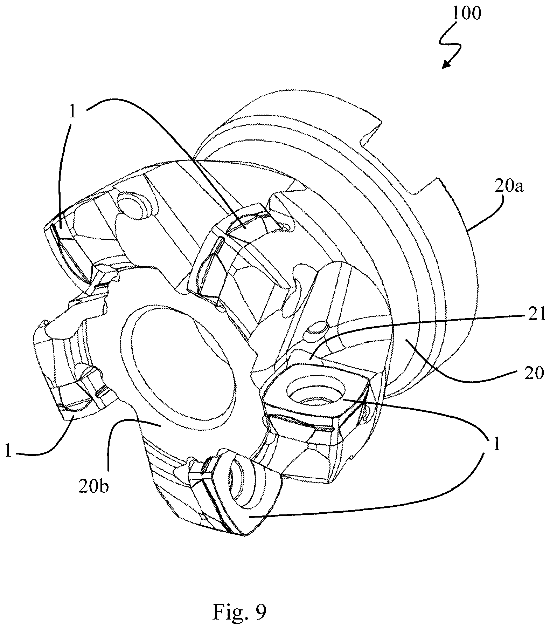

In Fig. 9 ist schematisch ein Fräswerkzeug 100 mit einer Mehrzahl der zuvor beschriebenen indexierbaren Schneideinsätze 1 dargestellt. Obwohl in Fig. 9 beispielhaft ein Fräswerkzeug 100 mit insgesamt fünf solchen indexierbaren Schneideinsätzen 1 gezeigt ist, sind auch Realisierungen möglich, bei denen das Fräswerkzeug weniger Schneideinsätze 1 oder mehr Schneideinsätze 1 aufweist.

Das Fräswerkzeug 100 ist zum Planfräsen mit großen Vorschüben ausgebildet und weist einen Halter 20 auf, dessen eines Ende 20a zur Verbindung mit einer Antriebswelle einer Fräsmaschine ausgebildet ist. Das Fräswerkzeug 100 ist dazu ausgebildet, zu einer rotierenden Bewegung um eine Rotationsachse angetrieben zu werden. An dem freien Ende 20b des Halters 20 ist eine Mehrzahl von Schneideinsatzaufnahmen 21 zur Aufnahme jeweils eines zuvor beschriebenen indexierbaren Schneideinsatzes 1 ausgebildet. Die Schneideinsatzaufnahmen 21 weisen jeweils eine Hauptauflagefläche zum Abstützen der Unterseite 3 des Schneideinsatzes 1 sowie zumindest eine Seitenauflagefläche, bevorzugt zwei Seitenauflageflächen, zum Abstützen der als Anlageflächen dienenden ersten Sekundärflächen 4b des Schneideinsatzes 1 auf. In der Hauptauflagefläche ist dabei eine Gewindebohrung zum Aufnehmen eines Gewindeabschnitts einer durch das Durchgangsloch 7 des Schneideinsatzes 1 geführten Befestigungsschraube ausgebildet.

Die Schneideinsatzaufnahmen 21 sind dabei derart ausgebildet, dass die in diesen befestigten Schneideinsätze 1 sowohl in axialer Richtung als auch in radialer Richtung von dem Halter 20 hervorstehen. Die Ausrichtung der Hauptauflageflächen und Seitenauflageflächen der Schneideinsatzaufnahmen 21 bestimmt dabei die effektiv realisierten Spanwinkel und Freiwinkel bei dem Fräsvorgang. Bei der dargestellten Ausführungsform sind die Schneideinsatzaufnahmen 21 gleichmäßig über den Außenumfang des Halters 20 verteilt angeordnet und weisen dabei jeweils übereinstimmende Orientierungen der Hauptauflageflächen und Seitenanlageflächen auf.

Die jeweilige Schneideinsatzaufnahme 21 ist dabei derart ausgebildet, dass ein darin befestigter indexierbarer Schneideinsatz 1 mit einer Schneidkante 5, die als aktive Schneidkante bezeichnet werden kann, in axialer Richtung über den Halter 20 hervorsteht. Diese aktive Schneidkante 5 ist dabei derart orientiert, dass ein Winkel zwischen der Schneidkante 5 und einer zu der Rotationsachse des Fräswerkzeugs 100 senkrechten Ebene entlang der Schneidkante 5 in radialer Richtung nach außen zunimmt. Ein radial innenliegender Bereich der Schneidkante 5 ist im Wesentlichen senkrecht zu der Rotationsachse des Fräswerkzeugs 100 orientiert.

## Patentansprüche

1. Indexierbarer Schneideinsatz (1) zum Planfräsen mit großen Vorschüben mit:
einer Oberseite (2),
einer Unterseite (3), die einen geringeren Außenumfang als die Oberseite (2) aufweist, und
die Oberseite (2) und die Unterseite (3) verbindenden Seitenflächen (4), wobei an dem Übergang zwischen den Seitenflächen (4) und der Oberseite (2) gerundete Schneidecken (6) ausgebildet sind, die über konvex ausgewölbte Schneidkanten (5) verbunden sind, die jeweils von einer Schneidecke (6) bis zu einer benachbarten Schneidecke (6) konvex gekrümmt verlaufen,
wobei die Seitenflächen (4) angrenzend an die Schneidkanten (5) jeweils Hauptfreiflächen (4a) aufweisen, die sich entlang der jeweiligen Schneidkante (5) durchgehend konvex gekrümmt von einer Schneidecke (6) bis zu einer benachbarten Schneidecke (6) erstrecken, die Seitenflächen (4) in allen Bereichen in Richtung zu der Unterseite (3) jeweils nach innen geneigt verlaufen, und
sich die Hauptfreiflächen (4a) in Richtung zu der Unterseite (3) nur über einen Teil der Höhe der Seitenflächen (4) erstrecken, **dadurch gekennzeichnet, dass** in Richtung zu der Unterseite (3), die Hauptfreiflächen (4a) in Form einer zu dem Zentrum des Schneideinsatzes (1) rückspringenden Stufe (9) gestuft in nach innen rückversetzte Sekundärflächen (4b, 4c) übergehen.

2. Indexierbarer Schneideinsatz nach Anspruch 1, wobei der Schneideinsatz eine in Aufsicht auf die Oberseite (2) im Wesentlichen quadratische Grundform mit vier Schneidecken (6) und vier die Schneidecken (6) verbindenden Schneidkanten (5) aufweist.

3. Indexierbarer Schneideinsatz nach Anspruch 1 oder 2, wobei sich die Hauptfreifläche (4a) in Richtung zu der Unterseite (3) über weniger als 50 % der Höhe der Seitenfläche (4) erstreckt, bevorzugt über weniger als 40 % der Höhe der Seitenfläche (4).

4. Indexierbarer Schneideinsatz nach einem der vorangehenden Ansprüche, wobei die Schneidkanten (5) einen im Wesentlichen ellipsensegment-förmigen Verlauf aufweisen.

5. Indexierbarer Schneideinsatz nach einem der vorangehenden Ansprüche, wobei entlang der Schneidkanten (5) und Schneidecken (6) an der Oberseite (2) eine Fase (8) ausgebildet ist.

6. Indexierbarer Schneideinsatz nach Anspruch 5, wobei die Fase (8) eine Breite zwischen 0,1 mm und 0,4 mm aufweist.

7. Indexierbarer Schneideinsatz nach Anspruch 5 oder 6, wobei die Fase (8) im Bereich der Schneideecke (6) unter einem ersten Fasenwinkel im Bereich zwischen -10° und -20° zu einer Haupterstreckungsrichtung der Unterseite (3) verläuft und im Bereich der Mitte der Schneidkante (5) unter einem größeren, zweiten Fasenwinkel zwischen 0° und -10°.

8. Indexierbarer Schneideinsatz nach Anspruch 7, wobei ein an die Schneidecke (6) angrenzender Bereich der Schneidkante (5) als ein Übergangsbereich (5a) ausgebildet ist, in dem der Winkel der Fase (8) von dem ersten Fasenwinkel auf den zweiten Fasenwinkel übergeht.

9. Indexierbarer Schneideinsatz nach einem der vorangehenden Ansprüche, wobei die Seitenflächen (4) über ihre gesamte Höhe unter einem schneideinsatzspezifischen Freiwinkel von ≥ 0° ausgebildet sind.

10. Indexierbarer Schneideinsatz nach einem der vorangehenden Ansprüche, wobei die Unterseite (3) als eine ebene Auflagefläche ausgebildet ist.

11. Indexierbarer Schneideinsatz nach einem der vorangehenden Ansprüche, wobei zumindest ein Teil der Sekundärflächen (4b, 4c) zumindest bereichsweise als ebene Anlageflächen ausgebildet ist.

12. Indexierbarer Schneideinsatz nach einem der vorangehenden Ansprüche mit einem den Schneideinsatz (1) von der Oberseite (2) zu der Unterseite (3) durchdringenden Durchgangsloch (7) zum Aufnehmen einer Befestigungsschraube.

13. Indexierbarer Schneideinsatz nach einem der vorangehenden Ansprüche, wobei der Schneideinsatz (1) in einem pulvermetallurgischen Herstellungsprozess gefertigt ist.

14. Indexierbarer Schneideinsatz nach einem der vorangehenden Ansprüche, wobei die Oberseite (2) mit zumindest einer Spanleitstufe versehen ist.

15. Fräswerkzeug (100) zum Planfräsen mit großen Vorschüben, mit:
einem Halter (20) mit einer Mehrzahl von Schneideinsatzaufnahmen (21) und
einer Mehrzahl von an den Schneideinsatzaufnahmen (21) befestigten Schneideinsätzen (1) nach einem der Ansprüche 1 bis 14.

## Claims

1. An indexable cutting insert (1) for face milling with high feed rates, comprising:
an upper side (2),
a lower side (3) which has a smaller outer circumference than the upper side (2), and
side surfaces (4) connecting the upper side (2) and the lower side (3), wherein, at the transition between the side surfaces (4) and the upper side (2), rounded cutting corners (6) are formed, which cutting corners are connected via convexly arched cutting edges (5) which each run in a convexly curved manner from one cutting corner (6) to an adjacent cutting corner (6),
wherein, adjacent to the cutting edges (5), the side surfaces (4) each have main flanks (4a) which extend along the respective cutting edge (5) in a continuously convexly curved manner from one cutting corner (6) to an adjacent cutting corner (6),
wherein the side surfaces (4) each run in an inwardly inclined manner in all regions in the direction of the lower side (3),
and
the main flanks (4a) extend in the direction of the lower side (3) only over part of the height of the side surfaces (4),
**characterized in that** in the direction of the lower side (3) the main flanks (4a) merge in the form of a step (9) recessed with respect to the center of the cutting insert (1) in a stepped manner into secondary surfaces (4b, 4c) which are set back inward.

2. The indexable cutting insert as claimed in claim 1, wherein the cutting insert has a basic shape which is substantially quadratic in a top view of the upper side (2), with four cutting corners (6) and four cutting edges (5) connecting the cutting corners (6).

3. The indexable cutting insert as claimed in claim 1 or 2, wherein the main flank (4a) extends in the direction of the lower side (3) over less than 50% of the height of the side surface (4), preferably over less than 40% of the height of the side surface (4).

4. The indexable cutting insert as claimed in one of the preceding claims, wherein the cutting edges (5) have a profile substantially in the shape of a segment of an ellipse.

5. The indexable cutting insert as claimed in one of the preceding claims, wherein a bevel (8) is formed along the cutting edges (5) and cutting corners (6) on the upper side (2).

6. The indexable cutting insert as claimed in claim 5, wherein the bevel (8) has a width between 0.1 mm and 0.4 mm.

7. The indexable cutting insert as claimed in claim 5 or 6, wherein the bevel (8) runs in the region of the cutting corner (6) at a first bevel angle within the range of between -10° and -20° with respect to a main direction of extent of the lower side (3) and in the region of the center of the cutting edge (5) at a larger second bevel angle of between 0° and -10°.

8. The indexable cutting insert as claimed in claim 7, wherein a region of the cutting edge (5) that is adjacent to the cutting corner (6) is designed as a transition region (5a) in which the angle of the bevel (8) merges from the first bevel angle into the second bevel angle.

9. The indexable cutting insert as claimed in one of the preceding claims, wherein the side surfaces (4) are formed over their entire height at a cutting-insert-specific clearance angle of ≥ 0°.

10. The indexable cutting insert as claimed in one of the preceding claims, wherein the lower side (3) is designed as a flat supporting surface.

11. The indexable cutting insert as claimed in one of the preceding claims, wherein at least part of the secondary surfaces (4b, 4c) is designed at least in regions as flat bearing surfaces.

12. The indexable cutting insert as claimed in one of the preceding claims, with a through hole (7) which penetrates the cutting insert (1) from the upper side (2) to the lower side (3) and is intended for receiving a fastening screw.

13. The indexable cutting insert as claimed in one of the preceding claims, wherein the cutting insert (1) is manufactured in a powder-metallurgical production process.

14. The indexable cutting insert as claimed in one of the preceding claims, wherein the upper side (2) is provided with at least one chip geometry.

15. A milling tool (100) for face milling with high feed rates, comprising:
a holder (20) with a plurality of cutting insert mounts (21), and
a plurality of cutting inserts (1) fastened to the cutting insert mounts (21) as claimed in one of claims 1 to 14.

## Revendications

1. Insert de coupe indexable (1) pour le surfaçage avec des avances importantes, comprenant :
un côté supérieur (2) ;
un côté inférieur (3), qui présente une circonférence extérieure plus petite que le côté supérieur (2), et
des surfaces latérales (4) reliant le côté supérieur (2) et le côté inférieur (3), des coins de coupe arrondis (6) étant formés au niveau de la transition entre les surfaces latérales (4) et le côté supérieur (2), qui sont reliés par des bords de coupe (5) bombés convexes qui s'étendent chacun de manière incurvée convexe d'un coin de coupe (6) à un coin de coupe (6) adjacent,
les surfaces latérales (4) présentant chacune, au voisinage des bords de coupe (5), des surfaces de dépouille principales (4a) qui s'étendent le long du bord de coupe (5) respectif de manière incurvée convexe continue d'un coin de coupe (6) à un coin de coupe (6) adjacent, les surfaces latérales (4) s'étendant chacune dans toutes les zones en direction du côté inférieur (3) de manière inclinée vers l'intérieur, et
les surfaces de dépouille principales (4a) s'étendant en direction du côté inférieur (3) uniquement sur une partie de la hauteur des surfaces latérales (4), **caractérisé en ce que**, dans la direction du côté inférieur (3), les surfaces de dépouille principales (4a) sont suivies par des surfaces secondaires (4b, 4c) évidées vers l'intérieur de manière échelonnée sous la forme d'un gradin (9) en retrait par rapport au centre de l'insert de coupe (1).

2. Insert de coupe indexable selon la revendication 1, dans lequel l'insert de coupe présente en vue de dessus sur le côté supérieur (2) une forme de base essentiellement carrée avec quatre coins de coupe (6) et quatre bords de coupe (5) reliant les coins de coupe (6) .

3. Insert de coupe indexable selon la revendication 1 ou 2, dans lequel la surface de dépouille principale (4a) s'étend en direction du côté inférieur (3) sur moins de 50 % de la hauteur de la surface latérale (4), de préférence sur moins de 40 % de la hauteur de la surface latérale (4).

4. Insert de coupe indexable selon l'une quelconque des revendications précédentes, dans lequel les bords de coupe (5) présentent une étendue essentiellement en forme de segment d'ellipse.

5. Insert de coupe indexable selon l'une quelconque des revendications précédentes, dans lequel un chanfrein (8) est formé le long des bords de coupe (5) et des coins de coupe (6) sur le côté supérieur (2).

6. Insert de coupe indexable selon la revendication 5, dans lequel le chanfrein (8) présente une largeur comprise entre 0,1 mm et 0,4 mm.

7. Insert de coupe indexable selon la revendication 5 ou 6, dans lequel le chanfrein (8) s'étend dans la zone des coins de coupe (6) à un premier angle de chanfrein dans la plage comprise entre -10° et -20° par rapport à une direction d'étendue principale du côté inférieur (3) et, dans la zone du milieu du bord de coupe (5), à un deuxième angle de chanfrein plus grand compris entre 0° et -10°.

8. Insert de coupe indexable selon la revendication 7, dans lequel une zone du bord de coupe (5) voisine des coins de coupe (6) est configurée en tant que zone de transition (5a), dans laquelle l'angle du chanfrein (8) passe du premier angle de chanfrein au deuxième angle de chanfrein.

9. Insert de coupe indexable selon l'une quelconque des revendications précédentes, dans lequel les surfaces latérales (4) sont configurées sur leur hauteur totale à un angle de dépouille spécifique à l'insert de coupe de ≥ 0°.

10. Insert de coupe indexable selon l'une quelconque des revendications précédentes, dans lequel le côté inférieur (3) est configuré en tant que surface d'appui plane.

11. Insert de coupe indexable selon l'une quelconque des revendications précédentes, dans lequel au moins une partie des surfaces secondaires (4b, 4c) sont configurées au moins en zones en tant que surfaces d'appui planes.

12. Insert de coupe indexable selon l'une quelconque des revendications précédentes, comprenant un trou de passage (7) traversant l'insert de coupe (1) du côté supérieur (2) au côté inférieur (3) pour la réception d'une vis de fixation.

13. Insert de coupe indexable selon l'une quelconque des revendications précédentes, dans lequel l'insert de coupe (1) est fabriqué dans un processus de production de métallurgie des poudres.

14. Insert de coupe indexable selon l'une quelconque des revendications précédentes, dans lequel le côté supérieur (2) est muni d'au moins un brise-copeau.

15. Outil de fraisage (100) pour le surfaçage avec des avances importantes, comprenant :
un support (20) muni d'une pluralité de logements d'insert de coupe (21), et
une pluralité d'inserts de coupe (1) selon l'une quelconque des revendications 1 à 14 fixés aux logements d'insert de coupe (21).
